# EUROPEAN PATENT APPLICATION

(11) **EP 4 810 080 A1**
(43) Date of publication of application: **23.09.2026**
(21) Application number: 25164299.7
(22) Date of filing: 18.03.2025
(51) Int. Cl.: A61M 5/142

(54) **INFUSION DEVICE**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application relates to an application-type infusion device, which comprises a casing body, a storage and infusion mechanism, a needle holder, an injection needle, an operating unit, an elastic part and a first guiding part, wherein an accommodating cavity is formed in the casing body, a distal end surface of the casing body that is close to the skin of the user is used to affix to the skin surface of the user, the storage and infusion mechanism is used to store and deliver a drug liquid to the injection needle, the first guiding part is configured to extend between a distal end direction close to the skin of the user and a proximal end direction facing away from the skin of the user, the needle holder sleeves over the periphery of the first guiding part, and the elastic part is disposed between the casing body and the needle holder. According to the present application, under the elastic restoring force applied by the elastic part, the needle holder may automatically remove itself together with the injection needle from the skin with convenient and rapid removal operation. In addition, the needle holder drives the injection needle to move between the distal end direction and the proximal end direction, so the injection needle is capable of being removed perpendicularly to the skin surface, thus preventing secondary damage to the skin.

## Description

### TECHNICAL FIELD

The present application relates to the field of medical devices, and particularly to an application-type infusion device.

### BACKGROUND

An application-type infusion device is configured to affixed on the skin surface of the user, for example, waist, arms and similar areas. After the injection needle is operated to insert into the skin, the drug liquid such as insulin stored in the infusion device is delivered to the user through the injection needle by means of a pump, and then the injection needle is removed from the skin surface after use. The application-type infusion device of the prior art needs to be removed with the aid of auxiliary tools, the removal process is complicated, and the injection needle is easy to cause secondary damage to the skin of the user during the removal process.

### SUMMARY

The present application provides an application-type infusion device.

Specifically, the present application is achieved through the following technical solution:
The present application provides an application-type infusion device, which comprises a casing body, a storage and infusion mechanism, a needle holder, an injection needle, an operating unit, an elastic part and a first guiding part, wherein an accommodating cavity is formed in the casing body, a distal end surface of the casing body that is close to the skin of the user is used to affix to the skin surface of the user, the storage and infusion mechanism is used to store and deliver a drug liquid to the injection needle, the first guiding part is configured to extend between a distal end direction close to the skin of the user and a proximal end direction facing away from the skin of the user, the needle holder sleeves over the periphery of the first guiding part, and the elastic part is disposed between the casing body and the needle holder, so that when the needle holder is driven by the operating unit to feed the injection needle in the distal end direction, the elastic part is gradually forced to store elastic energy; when the needle holder is locked between the distal end direction and the proximal end direction by the operating unit, the elastic part is configured into an elastic storage state; and when the needle holder is unlocked between the distal end direction and the proximal end direction by the operating unit, the elastic part gradually releases the elastic restoring force to drive the needle holder to retract the injection needle in the proximal end direction.

In some embodiments, a proximal end of the first guiding part is provided with a stopper.

In some embodiments, a distal end of a guide part is connected to the casing body. In some embodiments, the casing body is configured in a split type, and application back glue for affixing to the skin surface of the user is applied on a surface of the casing body that is close to the distal end.

In some embodiments, the elastic part is a coil spring disposed between the needle holder and a part of the casing body that is close to the distal end, and the coil spring sleeves over the periphery of the first guiding part.

In some embodiments, the needle holder sleeves the first guiding part in a middle area in a horizontal direction parallel to the skin surface of the user, a notch is formed on at least two adjacent sides of the needle holder in the horizontal direction, separately, and the application-type infusion device is also provided with a second guiding part, which extends between the distal end direction and the proximal end direction and is embedded in the notch with a matching shape. In some embodiments, a groove is formed in the needle holder and extends in the horizontal direction parallel to the skin surface of the user, and a hose for connecting the storage and infusion mechanism with the injection needle passes through the groove and is connected to the injection needle.

In some embodiments, the operating unit is provided with an operating part and a pushing part, the pushing part initially extends from the operating part to the distal end in a cylindrical shape, and a locking structure that is mutually matched and staggered in a circumferential direction is arranged between the cylindrical periphery of the pushing part and the casing body, so as to be suitable for screwing the operating unit relative to the casing body when the operating unit and the casing body are locked between the distal end direction and the proximal end direction by means of the locking structure, thereby achieving unlocking between the operating unit and the casing body.

In some embodiments, a first claw radially extending outward is disposed at the cylindrical periphery of the pushing part, the casing body is provided with a snap ring radially extending inward at the periphery of the operating unit, and the snap ring is configured with a first slot which at least coincides with the profile of the first claw, so that the first claw may cooperate with the snap ring to achieve locking, and the first claw may cooperate with the first slot to achieve unlocking. In some embodiments, the cylindrical periphery of the pushing part is provided with a convex strip extending between the distal end direction and the proximal end direction, the snap ring is configured with a second slot which at least coincides with the profile of the convex strip, and a gap which the snap ring fits into is formed between the convex strip and the operating part, so that the snap ring may pass through the gap, thereby the operating unit is allowed to be screwed relative to the casing body until the first claw is aligned with the first slot. According to the present application, affixing the distal end surface of the casing body to the skin surface of the user, and pressing the operating unit between the distal end direction and the proximal end direction may drive the needle holder to force the injection needle into the skin, and the injection needle is capable of being locked in the skin by means of the operating unit to deliver the drug liquid. When the infusion device needs to be removed, the needle holder is unlocked through the operating unit, and then the needle holder may be automatically removed from the skin together with the injection needle under the elastic restoring force applied by the elastic part. The removal operation is convenient and rapid. In addition, the needle holder drives the injection needle to move between the distal end direction and the proximal end direction, so the injection needle is capable of being removed perpendicularly to the skin surface, thus preventing secondary damage to the skin.

It is understood that the foregoing general description and the description in the following paragraphs are merely exemplary and explanatory, and are not intended to limit the present application.

### BRIEF DESCRIPTION OF DRAWINGS

The drawings herein, which are incorporated into the Description and form a part of the Description, show the embodiments complying with the present application, and are used together with the Description to explain the principles of the present application.
FIG. 1 is a schematic diagram of an application-type infusion device in one embodiment of the present application;
FIG. 2 is a first exploded view of the application-type infusion device in one embodiment of the present application;
FIG. 3 is a second exploded view of the application-type infusion device in one embodiment of the present application;
FIG. 4 is a sectional view of the removed application-type infusion device in one embodiment of the present application;
FIG. 5 is a sectional view of the locked application-type infusion device in one embodiment of the present application; and
FIG. 6 is a sectional view of the unlocked application-type infusion device in one embodiment of the present application.

Reference numerals:
10: operating unit; 11: operating part; 12: pushing part; 13: first claw; 14: convex strip; 15: gap; 16: second claw;
21: top casing; 211: snap ring; 22: bottom casing; 221: second guiding part; 222: first slot; 223: second slot; 224: partition panel; 225: stiffening plate; 23: application back glue;
30: storage and infusion mechanism;
40: needle holder; 41: injection needle; 42: notch; 43: groove;
50: first guiding part; 51: stopper;
60: elastic part;
70: hose.

### DETAILED DESCRIPTION OF THE EMBODIMENTS

The present application will now be discussed with reference to several embodiments. It should be understood that these embodiments are discussed only to enable those skilled in the art to better understand and thus realize the present application, and are not intended to imply any limitation on the scope of the present application.

As used herein, the term "comprise" and variations thereof should be interpreted as open-class terms meaning "comprise but not limited to"; the terms "embodiment" and "one embodiment" should be interpreted as "at least one embodiment"; the term "another embodiment" should be interpreted as "at least one other embodiment"; the terms "first" and "second" may refer to different or the same object; and the term "dispose" is not limited to direct connection or indirect connection, nor is it limited to a specific connection mode. Other explicit and implicit definitions may be set forth in the following text.

Some specific values or numerical ranges may be involved in the following description. It should be understood that these values and numerical ranges are merely exemplary, which may be helpful for putting the ideas of the present application into practice. However, the description of these embodiments is not intended to limit the scope of the present application in any way. According to specific application scenarios and requirements, these values or numerical ranges are able to be set additionally.

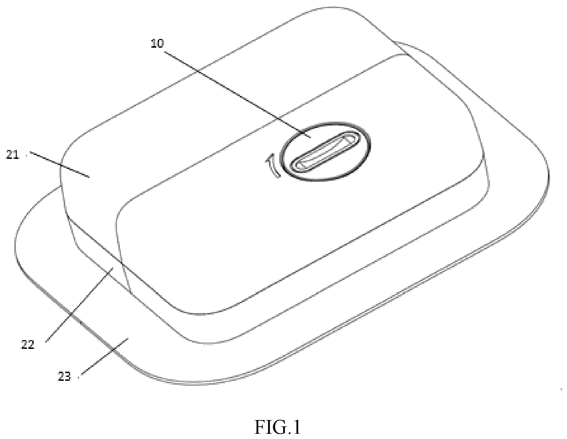

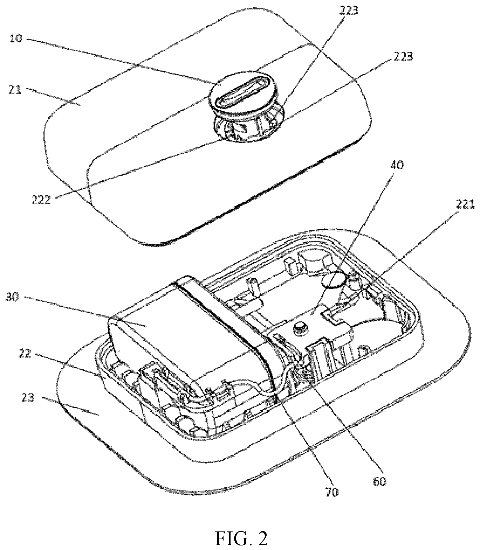

As mentioned above, the application-type infusion device of the prior art needs to be removed with the aid of auxiliary tools, the removal process is complicated, and the injection needle is easy to cause secondary damage to the skin of the user during the removal process. An application-type infusion device proposed by the embodiment of the present application at least partially solves the above-mentioned problems. It should be noted that the application-type infusion device in the embodiment of the present application is not limited to delivering insulin, but also delivering other drug liquids, including but not limited to vitamins, glucose and normal saline. As shown in FIG. 1 to FIG. 6, the application-type infusion device in the embodiment of the present application generally comprises a casing body, a storage and infusion mechanism 30, a needle holder 40, an injection needle 41, an operating unit 10, an elastic part 60, a hose 70 and a first guiding part 50, wherein an accommodating cavity is formed in the casing body to accommodate all parts of the infusion device; the storage and infusion mechanism 30 is used to store a drug liquid and deliver the drug liquid to the injection needle 41 through the hose 70, the storage and infusion mechanism 30 may be provided with a storage container and a pump element, wherein the storage container is pre-filled with the drug liquid, and the pump element is powered by a battery or a power supply to pump out the drug liquid in the storage container; the injection needle 41 is fixedly disposed on the needle holder 40, and the needle holder 40 drives the injection needle 41 to move as a whole; the operating unit 10 is used by the user for pressing and screwing operations, and the needle holder 40 is driven to move by means of the operating unit 10; the first guiding part 50 is used to guide the needle holder 40 to move accurately in a preset direction; and the elastic part 60 drives the needle holder 40 to be removed from the skin by its own energy storage and release functions.

In order to understand the structure and working principle of the application-type infusion device in the embodiment of the present application, in the direction perpendicular to the skin surface of the user, a surface close to the skin surface is defined as a distal end, and a surface facing away from the skin surface is defined as a proximal end. According to the application-type infusion device in the embodiment of the present application, when the casing body is affixed on the skin surface, the needle holder 40 and the injection needle 41 are ensured to move accurately between the distal end and the proximal end.

The casing body in the embodiment of the present application comprises a top casing 21 and a bottom casing 22, the bottom casing 22 is close to the skin surface of the user, the top casing 21 faces away from the skin surface, and a step structure is formed at a splicing joint between the top casing 21 and the bottom casing 22, thereby improving the splicing tightness and firmness. The top casing 21 and the bottom casing 22 are spliced together to form the accommodating cavity, and the storage and infusion mechanism 30, the needle holder 40, the injection needle 41, the operating unit 10, the elastic part 60, the hose 70, a controller (not shown in the figures), the power supply (not shown in the figures) and the like are arranged in the accommodating cavity. Exemplarily, the power supply may be a battery. The application-type infusion device in the embodiment of the present application pushes the needle holder 40 by means of the operating unit 10 to drive the injection needle 41 to move to the distal end, thereby feeding the injection needle 41. During the needle feeding process, the elastic part 60 is gradually forced to store energy. When the injection needle 41 needs to be removed, the pressing force exerted on the operating unit 10 is removed, and the elastic part 60 releases energy and pushes the needle holder 40 to drive the injection needle 41 to move to the proximal end, thereby retracting the injection needle 41. In one embodiment, the elastic part 60 may be a coil spring as shown in the figure, which is disposed between the needle holder 40 and the bottom casing 22, and is compressed to store energy. In another embodiment, the elastic part 60 may also be a coil spring disposed between the needle holder 40 and the top casing 21, with both ends hooked on the needle holder 40 and the top casing 21 respectively, and the coil spring is stretched to store energy. In other embodiments, the elastic part 60 may also be an elastic sheet, which is bent to store energy.

The needle holder 40 is fixedly connected to the injection needle 41, one end of the hose 70 is communicated with the injection needle 41, and the other end of the hose 70 is communicated with a storage mechanism, and the storage mechanism delivers the drug liquid to the injection needle 41 through the hose 70. In one embodiment, a groove 43 is formed in the needle holder 40, and the width of the groove 43 is approximate to the diameter of the hose 70, so that the hose 70 is capable of being clamped in the groove 43, thereby preventing the hose 70 from disconnecting with the moving needle holder 40 and from winding. Exemplarily, the extending direction of the groove 43 may be consistent with the horizontal direction parallel to the skin surface, so that more space for arranging the hose 70 is available.

In order to ensure that the moving direction of the injection needle 41 driven by the needle holder 40 accurately coincides with the direction perpendicular to the skin surface, a first guiding part 50 and a second guiding part 221 extending between the distal end and the proximal end are arranged. In one embodiment, the first guiding part 50 passes through the middle area of the needle holder 40. When the operating unit 10 applies pressure to the needle holder 40, it ensures that the force is evenly transmitted to the first guiding part 50 through the needle holder 40, and prevents the needle holder 40 from locking with the first guiding part 50 due to inclination during movement. Exemplarily, the first guiding part 50 is a cylindrical pin, which only provides a radial limit of the cylindrical pin for the needle holder 40.

In one embodiment, the first guiding part 50 may freely pass through the needle holder 40; alternatively, one end of the first guiding part 50 close to the distal end is fixedly connected to the bottom casing 22. Exemplarily, one end of the first guiding part 50 is fixedly connected to the bottom casing 22 through threaded connection or welding. In another embodiment, the coil spring sleeves over the periphery of the first guiding part 50, and the first guiding part 50 provides additional stable support for the coil spring, so as to prevent the coil spring from deviating in the process of storing elastic energy and releasing the elastic restoring force.

The user pushes, locks, unlocks and releases the needle holder 40 and the injection needle 41 by pressing and screwing the operating unit 10. In one embodiment, the operating unit 10 is provided with an operating part 11 and a pushing part 12, the operating part 11 is used by the user for pressing and screwing, and the pushing part 12 initially extends from the operating part 11 to the distal end in a cylindrical shape, and pushes the needle holder 40 to move by means of the cylindrical distal end.

The cylindrical periphery is provided with a convex strip 14 extending between the distal end direction and the proximal end direction, the top casing 21 is provided with a snap ring 211, the snap ring 211 is configured with a second slot 223, and the second slot 223 has a profile and a circumferential position fitting with the convex strip 14, so as to provide circumferential limitation for the operating unit 10 when the operating unit 10 is moving under pressure and prevent the operating unit 10 from rotating.

A first claw 13 radially extending outward is disposed at the cylindrical periphery close to the operating part 11. When the operating unit 10 is pressed to move to a locking position, the first claw 13 is elastically compressed by the snap ring 211 in the radial direction and rebounds, and then the first claw 13 and the snap ring 211 are clamped, thus realizing mutual locking between the operating unit 10 and the top casing 21.

A gap 15 is formed at a position of the convex strip 14 close to the operating part 11, and the position where the gap 15 is formed corresponds to the first claw 13. Additionally, the snap ring 211 is also configured with a first slot 222, the first slot 222 has a profile fitting with the first claw 13 and a position corresponding to an unlocking twist angle, so that the operating unit 10 is allowed to be screwed when the first claw 13 is locked with the snap ring 211. In that case, the snap ring 211 may pass through the gap 15. When the first claw 13 is screwed to the position corresponding to that of the first slot 222, the user releases the pressure on the operating unit 10, and the elastic part 60 releases energy to push the first claw 13 to pass through the first slot 222, thus realizing the unlocking between the operating unit 10 and the top casing 21.

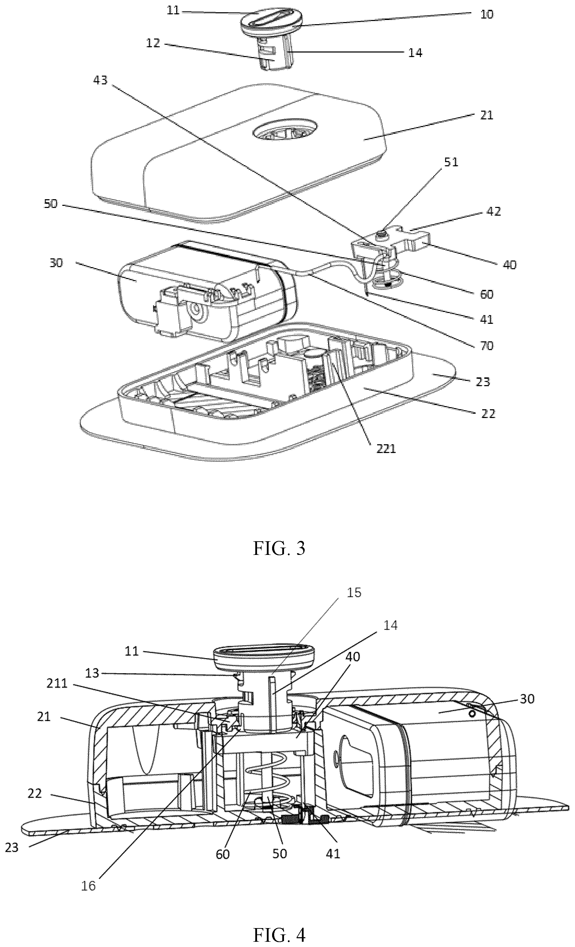

In one embodiment, a second claw 16 extending radially outward is disposed at the cylindrical periphery close to the distal end direction, and the second claw 16 and the first claw 13 are arranged in a staggered manner in the circumferential direction of the cylinder, so as to ensure that when the operating unit 10 is in the unlocked state, as shown in FIG. 4, the second claw 16 and the snap ring 211 are clamped to prevent the operating unit 10 from being disengaged from the top casing 21.

In one embodiment, one end of the first guiding part 50 that is close to the proximal end is provided with a stopper 51, and the stopper 51 is larger than an opening configured in the needle holder 40 for being passed through by the first guiding part 50, thereby preventing the first guiding part 50 from being disengaged from the needle holder 40.

In one embodiment, the second guiding part 221 is disposed at an edge area of the needle holder 40 in the horizontal direction, and the needle holder 40 has a notch 42 in the edge area. The notch 42 has a profile fitting with the second guiding part 221, so that the second guiding part 221 is capable of being embedded in the notch 42. The second guiding part 221 may also provide accurate guidance to the moving direction of the needle holder 40, and since the second guiding part 221 is disposed at the edge area of the needle holder 40, it may also provide the needle holder 40 with a circumferential rotation limitation around the first guiding part 50. Exemplarily, the profile of the second guiding part 221 and the notch 42 may be quadrangle or other polygons to further provide stable limitation.

In one embodiment, one or more second guiding parts 221 may be provided. As shown in the figures, totally two second guiding parts 221 are arranged, which are located in two mutually orthogonal edge areas of the needle holder 40, respectively. Such arrangement may provide circumferential limitation for the needle holder 40 from two orthogonal directions.

In one embodiment, the bottom casing 22 is also provided with a partition panel 224, and the storage mechanism and the needle holder 40 are arranged on either side of the partition panel 224. The partition panel 224 may lay against the side of the needle holder 40 without the notch 42, so as to further provide guidance and limitation for the needle holder 40.

In one embodiment, a stiffening plate 225 is disposed at the inner side of a peripheral wall of the bottom casing 22, thereby providing structural reinforcement to the peripheral wall. In another embodiment, the stiffening plate 225 (not shown in the figure) may also be disposed on the inner side of a peripheral wall of the top casing 21.

In one embodiment, application back glue 23 is applied on a surface of the bottom casing 22 that is close to the skin surface, which is convenient for feeding the injection needle 41 and retracting the injection needle 41 after affixing the casing body on the skin surface.

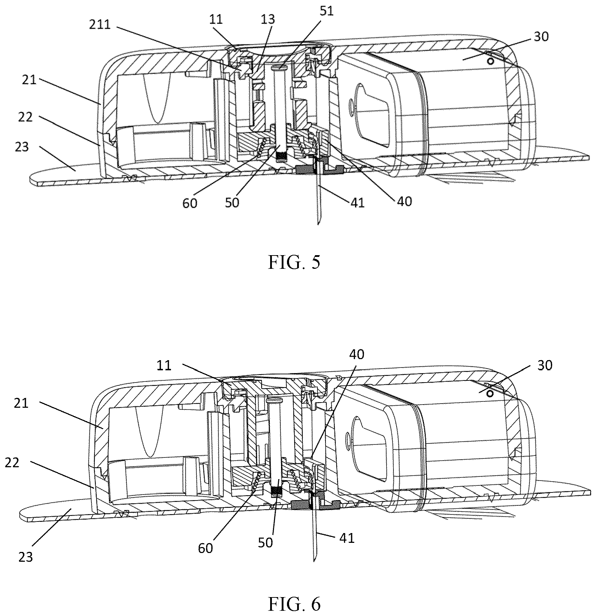

When using the application-type infusion device in the embodiment of the present application, firstly, the infusion device is firmly affixed on the skin surface of the user by means of the application back glue 23, and then the operating unit 10 is pressed to drive the needle holder 40 and the injection needle 41 to insert into the skin. As shown in FIG. 5, the operating unit 10 is locked with the top casing 21 through the first claw 13, and the storage and infusion mechanism 30 is enabled to deliver the drug liquid to the injection needle 41. When the drug liquid delivery is finished, the operating unit 10 is screwed to align the first claw 13 with the first slot 222, and align the convex strip 14 with the second slot 223 simultaneously to realize unlocking, as shown in FIG. 6. The operating unit 10 is automatically ejected by energy release of the elastic part 60. During the ejection process, the first claw 13 moves through the first slot 222 and the convex strip 14 moves through the second slot 223, thus driving the injection needle 41 to be removed from the skin, as shown in FIG. 4. The whole operation process is convenient and rapid, and will not cause secondary damage to the skin.

The description of the embodiments herein and any reference to the direction and orientation are only used for the convenience of description, and cannot be interpreted as any limitation on the scope of protection of the present application. The description of the preferred embodiments will involve the combination of features, which may exist independently or in combination. The present application is not particularly limited to the preferred embodiments. The scope of protection set forth by the present application is defined by the claims.

Only preferred embodiments of the present application are described above, but the present application is not limited thereto. Any modification, equivalent replacement and improvement made within the spirit and rule of the present application shall be incorporated in the scope of protection of the present application.

## Claims

1. An application-type infusion device, comprising a casing body, a storage and infusion mechanism, a needle holder, an injection needle, an operating unit, an elastic part and a first guiding part, wherein an accommodating cavity is formed in the casing body, a distal end surface of the casing body that is close to skin of the user is configured to affix to skin surface of the user, the storage and infusion mechanism is configured to store and deliver drug liquid to the injection needle, the first guiding part is configured to extend between a distal end direction close to the skin of the user and a proximal end direction facing away from the skin of the user, the needle holder sleeves over the periphery of the first guiding part, and the elastic part is disposed between the casing body and the needle holder, wherein when the needle holder is driven by the operating unit to feed the injection needle in the distal end direction, the elastic part is gradually forced to store elastic energy; when the needle holder is locked between the distal end direction and the proximal end direction by the operating unit, the elastic part is configured to enter into an elastic storage state; and when the needle holder is unlocked between the distal end direction and the proximal end direction by the operating unit, the elastic part gradually releases the elastic restoring force to drive the needle holder to retract the injection needle in the proximal end direction.

2. The application-type infusion device according to claim 1, wherein a proximal end of the first guiding part comprises a stopper.

3. The application-type infusion device according to claim 1, wherein a distal end of a guide part is connected to the casing body.

4. The application-type infusion device according to claim 1, wherein the casing body is configured in a split type, and application back glue for affixing to the skin surface of the user is applied on a surface of the casing body and is close to the distal end.

5. The application-type infusion device according to claim 1, wherein the elastic part is a coil spring disposed between the needle holder and a part of the casing body that is close to the distal end, and the coil spring sleeves over the periphery of the first guiding part.

6. The application-type infusion device according to claim 1, wherein the needle holder sleeves the first guiding part in a middle area in a horizontal direction parallel to the skin surface of the user, a notch is formed on at least two adjacent sides of the needle holder in the horizontal direction, separately, and the application-type infusion device further comprises a second guiding part, which extends between the distal end direction and the proximal end direction and is embedded in the notch with a matching shape.

7. The application-type infusion device according to claim 1, wherein a groove is formed in the needle holder and extends in the horizontal direction parallel to the skin surface of the user, and a hose for connecting the storage and infusion mechanism with the injection needle passes through the groove and is connected to the injection needle.

8. The application-type infusion device according to claim 1, wherein the operating unit comprises an operating part and a pushing part, the pushing part initially extends from the operating part to the distal end in a cylindrical shape, and a locking structure that is matched and staggered in a circumferential direction is arranged between the cylindrical periphery of the pushing part and the casing body, wherein screwing the operating unit relative to the casing body, when the operating unit and the casing body are locked between the distal end direction and the proximal end direction by means of the locking structure, unlocks the operating unit and the casing body.

9. The application-type infusion device according to claim 8, wherein a first claw radially extending outward is disposed at the cylindrical periphery of the pushing part, the casing body comprises a snap ring radially extending inward at the periphery of the operating unit, and the snap ring is configured with a first slot which at least coincides with the profile of the first claw, wherein the first claw is configured to cooperate with the snap ring to achieve locking, and the first claw is configured to cooperate with the first slot to achieve unlocking.

10. The application-type infusion device according to claim 9, wherein the cylindrical periphery of the pushing part comprises a convex strip extending between the distal end direction and the proximal end direction, the snap ring is configured with a second slot which at least coincides with the profile of the convex strip, and a gap which the snap ring fits into is formed between the convex strip and the operating part, wherein the snap ring is configured to pass through the gap, thereby the operating unit is allowed to be screwed relative to the casing body until the first claw is aligned with the first slot.
